# EUROPEAN PATENT APPLICATION

(11) **EP 2 584 483 A1**
(43) Date of publication of application: **24.04.2013**
(21) Application number: 11185411.3
(22) Date of filing: 17.10.2011
(51) Int. Cl.: G06F 19/00

(54) **Patient staff board system**

(71) Applicant: Koninklijke Philips Electronics N.V., 5621 BA Eindhoven (NL)
(72) Inventor: Cuppen, Roel, 5600 AE Eindhoven (NL); Flinsenberg, Ingrid, C., M., 5600 AE Eindhoven (NL); Loenen, van Evert, J., 5600 AE Eindhoven (NL); Daemen, Elke, M., L., 5600 AE Eindhoven (NL); Rajae-Joordens, Rosemarie, J., E., 5600 AE Eindhoven (NL)
(74) Representative: Damen, Daniel Martijn

(57) **Abstract**

The invention relates to a system (102) for providing to a patient (104) information concerning a medical professional entering an accompanying patient room (106). To that end the system (102) comprises a detector (108) configured for detecting an entry of the medical professional in the patient room (106). The system (102) furthermore comprises a computer (110) for retrieving the information concerning the medical professional from an organized collection of data (112) in response to detecting the entry of said medical professional in the patient room by the detector (108). Furthermore, the system (102) comprises an electronic display (114) for displaying to the patient (104) said information.

## Description

### FIELD OF THE INVENTION

The invention relates to a system for providing to a patient information concerning a medical professional entering an accompanying patient room.

### BACKGROUND OF THE INVENTION

During a hospital stay a patient will come across multiple medical professionals having various roles, such as nurses, doctors, therapists and social workers. Such medical professionals usually introduce themselves via mentioning their names and roles to the patient in order to have a comfortable and personal meeting. However, either because of information overload or because of the patient's health conditions, patient often cannot reproduce all names and roles. This causes patients to feel uncomfortable and at unease, both during their meetings with medical professionals and thereafter. This will induce some level of stress at the patient which stress has a negative impact on the patient's healing.

### SUMMARY OF THE INVENTION

It is an object of the invention to provide a system capable of reducing a patient's stress level associated with encountering a variety of medical professionals during a hospital stay. This object of the invention is achieved by the system according to the invention, which system is configured for providing to a patient information concerning a medical professional entering an accompanying patient room, which system comprises a detector configured for detecting an entry of the medical professional in the patient room, a computer for retrieving the information concerning the medical professional from an organized collection of data in response to detecting the entry of said medical professional in the patient room by the detector, and an electronic display for displaying to the patient said information.

By detecting an entry of the medical professional in the patient room via the detector, by executing via a computer a query language to retrieve information from a database concerning the medical professional as detected and by subsequently displaying the information thus retrieved via an electronic display, information concerning the medical professional is visually available for the patient right from the start of the visit paid by said medical professional. As a result, the patient is enabled to look up during the visit information concerning the medical professional in a convenient way. Hence the system according to the invention removes a cause for discomfort and unease. Therefore the system according to the invention is effectively capable of reducing the patient's stress level associated with encountering a variety of medical professionals during his or her hospital stay.

The system according to the invention furthermore has the advantage in that it provides information very legibly by way of the electronic display, as opposed to state of the art wearable name tags or badges.

In a preferred embodiment of the system according to the invention, the detector is furthermore configured for detecting an exit of the medical professional from the patient room, and wherein the electronic display is configured for ceasing displaying the information concerning said medical professional in response to detecting the exit of said medical professional from the patient room by the detector. By detecting the exit of the medical professional and by subsequently ceasing the display of information concerning said professional via the electronic display, this embodiment enables providing information to the patient in respect of the medical professional actually present in the patient room. Thereby this embodiment prevents from mixing up information relating to various medical professionals which may be a source of stress for the patient. For that reason this embodiment advantageously further reduces a patient's stress level associated with the range of visits by a variety of medical professionals during a hospital stay.

In a further preferred embodiment of the system according to the invention, the electronic display is configured for simultaneously displaying information concerning a plurality of medical professionals in response to detecting the entries of said plurality of medical professionals in the patient room by the detector. During a visit by multiple medical professionals, the chance of information overload is even larger and relevant information concerning the medical professionals is even more difficult to grasp. Hence, during such visits patients may feel even more uncomfortable. This embodiment makes visually available for the patient, right from the points in time the various medical professionals enter, information concerning these medical professionals. Therefore this embodiment is advantageous in that it further reduces a patient's stress level associated with visits by medical professionals during his or her hospital stay.

In a further preferred embodiment of the system according to the invention, the electronic display is configured for displaying information concerning the plurality of medical professionals in an arrangement according to an order of detecting the entries of said plurality of medical professionals in the patient room by the detector. This embodiment enables a patient to link part of the information made available via the display to one of the medical professionals present in the patient room. Therefore this embodiment improves putting the information concerning the plurality of medical professionals in its right context. Consequently, this embodiment has the advantage of further reducing the patient's stress level relating to meetings with medical professionals during a hospital stay.

In a practical embodiment of the system according to the invention, the electronic display is configured for displaying the name of the medical professional.

In a further practical embodiment of the system according to the invention, the electronic display is configured for displaying information relating to the role of the medical professional.

In a further preferred embodiment of the system according to the invention, the electronic display is configured for displaying a photograph of the medical professional. This embodiment makes available the information concerning the medical professional in a way that is beneficial for patients having limited ability to read e.g. children or neurology patients. Therefore, this embodiment advantageously further reduces stress levels for those kinds of patients.

A further preferred embodiment of the system according to the invention comprises a data storage for storing the information concerning the medical professional, and a user interface for accessing the information stored by the data storage. This embodiment enables both a patient and its relatives to retrieve information concerning the medical professionals at some later point in time, thereby gaining an overview of the visits paid by various medical professionals. Hence, this embodiment circumvents the need for the patient to actually remember all information displayed to him or her via the display. As a result this embodiment is advantageous in that it further reduces the patient's stress level relating to meetings with medical professionals during a hospital stay.

In a further preferred embodiment of the system according to the invention, the data storage is configured for storing a timestamp along with the information concerning the medical professional. This embodiment advantageously allows the patient and/or its relatives to gain an ever more detailed overview, i.e. including an indication of time, regarding the various encounters with medical professionals.

### BRIEF DESCRIPTION OF THE DRAWINGS

Fig. 1 schematically display an embodiment of the system embedded in and around a patient room.
Figs. 2A, 2B, 2C and 2D schematically depict an embodiment of the system in which the electronic display is configured for displaying information concerning various medical professionals in an arrangement according to their entry in the patient room.

### DETAILED DESCRIPTION OF THE EMBODIMENTS

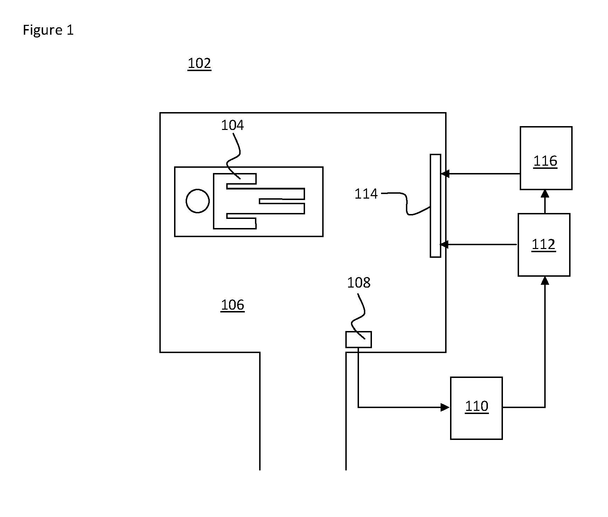

Figure 1 schematically displays a view from above for a system 102 for providing to a patient 104 information concerning a medical professional entering an accompanying patient room 106 i.e. the room in which the patient 104 resides. The system 102 comprises a detector 108 known per se configured for detecting an entry of the medical professional in the patient room 106. The detector may, without limitation, employ a technology comprising a transmitter for transmitting some signal, e.g. electromagnetic radiation, and a reader for receiving said signal. The reader may be fixed and situated near or in the doorway of the patient room 106 whereas the transmitter may be worn by the medical professional. Alternatively, the transmitter may be worn by the medical professional whereas the reader is fixed and situated near or in the doorway of the patient room 106. More specifically, the detector 108 may employ radio-frequency identification (RFID) in order to detect the medical professional. For this purpose the medical professional is to carry an RFID tag or label which is to be read by an RFID reader. Herein either passive RFID or active RFID, in which the tag or label permanently broadcasts or beacons a signal, may be employed. Alternatively, the detector may employ a technology based on transmission of infrared, ultrasound or other beacon between some transmitter and some reader. Furthermore, the detector 108 may employ camera vision technology.

Preferably, as indicated in Figure 1, the detector 108, e.g. aforementioned reader, transmitter or camera, is situated near a doorway 110 of the patient room 106 as to prevent from false positive indications regarding entry of the medical professional. More preferably, the detector 108 is situated inside said patient room 106. In order to provide the patient with the information concerning the medical professional more in advance of the physical entry of said professional in the patient room 106, the detector 108 may be situated more remote from the patient room 106.

The system 102 furthermore comprises a computer 110 for retrieving the information, e.g. by executing a query language, concerning the medical professional from an organized collection of data 112 in response to detecting the entry of said medical professional in the patient room 106 by the detector 108. The organized collection of data 112 may for example be a database. In order to retrieve information from such database, the computer 110 is configured for executing a query language. The system 102 furthermore comprises an electronic display (114) for displaying to the patient 104 said information. The electronic display 114 may be a display known per se such as an LED or a plasma monitor. The computer 110 may be situated at some convenient place; there is no need to situate it as near to the patient room 106 as displayed in Figure 1.

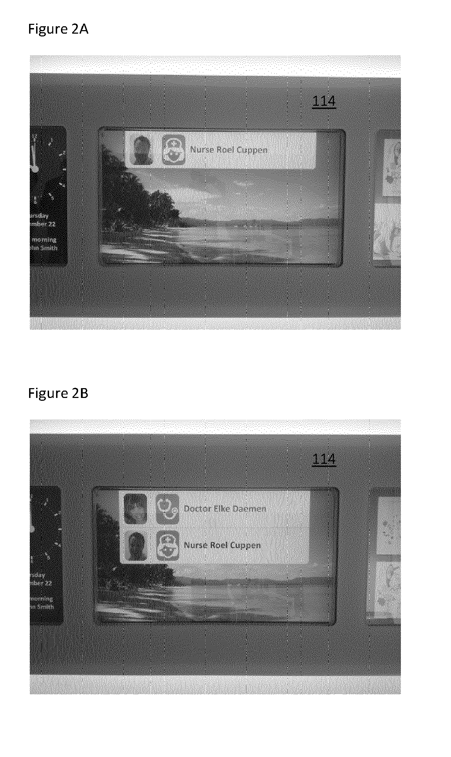

Referring to Figure 2A, in this specific example the electronic display 114 is arranged for displaying the name the medical professional whose entry is detected by the detector 108, the role of said medical professional, in this particular case a nurse, and a photograph of said medical professional.

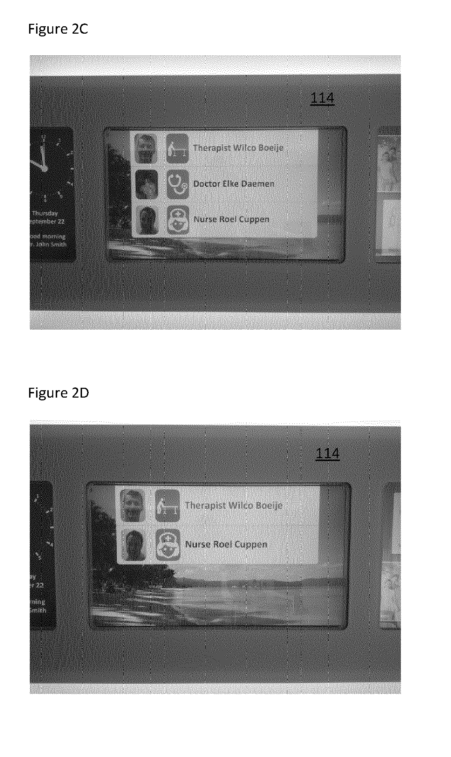

Referring to Figure 2B, in this particular embodiment the electronic display 114 is configured for simultaneously displaying information concerning a plurality of medical professionals in response to detecting the entries of said plurality of medical professionals in the patient room 106 by the detector 108. For example, after detection of the entry of a nurse by the detector 108, information concerning a doctor may be displayed simultaneously in response to detecting the entry of said doctor by the detector 108. Furthermore, referring to Figure 2C, in response to detecting the entry of a therapist by the detector, information concerning said therapist is displayed by the display 114 as well.

Referring to both Figure 2B and Figure 2C, in this specific embodiment the electronic display 14 is furthermore configured for displaying information concerning the plurality of medical professionals in an arrangement according to an order of detecting the entries of said plurality of medical professionals in the patient room 106 by the detector 108. That is, as the nurse was detected first by the detector 108, the doctor second and the therapist thereafter, the information concerning these medical professionals is displayed in said order. In this particular example, information concerning the medical professional whose entry is most recently detected by the detector 108, is put on top of the information already being displayed at the electronic display 114.

In this specific embodiment the detector 108 is furthermore configured for detecting an exit of the medical professional from the patient room 106 and the electronic display 114 is configured for ceasing displaying the information concerning said medical professional in response to detecting the exit of said medical professional from the patient room 106 by the detector 108. Alternative technologies may be employed by the detector 108 for distinguishing between entry and exit. For example, if the detector 108 employs passive RFID, a first reading by the reader implies an entry of the medical professional in the patient room whereas a second reading by the reader must indicate an exit of said medical professional. More generally, an odd reading indicates an entry and an even reading implies an exit. Alternatively, if the detector 108 employs active RFID, as long as the signal being beaconed by the badge is being received by the reader, the medical professional is present in the patient room 106. Hence, a start of said signal receiving indicates entering whereas an end of said receiving denotes exiting. If the detector 108 were to employ camera vision technology, distinguishing between entry and exit follows from the content of the camera images. Hence, referring to Figure 2D, if for example the detector 108 during operation detects that the doctor exits the patient room 106, information concerning said doctor is no longer displayed at the display 114 in response thereto.

In this particular example, the system 102 furthermore comprises a data storage 116 known per se for storing the information concerning the medical professional, and a user interface known per se for accessing the information stored by the data storage. Preferable, the use interface is integrated within the electronic display 114. Furthermore, in this specific embodiment, the data storage 116 is configured for storing a timestamp along with the information concerning the medical professional.

While the invention has been illustrated and described in detail in the drawings and in the foregoing description, the illustrations and the description are to be considered illustrative or exemplary and not restrictive. The invention is not limited to the disclosed embodiments. Other combinations of embodiments are feasible. It is noted that the system according to the invention and all its components can be made by applying processes and materials known per se. In the set of claims and the description the word "comprising" does not exclude other elements and the indefinite article "a" or "an" does not exclude a plurality. Any reference signs in the claims should not be construed as limiting the scope. It is further noted that all possible combinations of features as defined in the set of claims are part of the invention.

## Claims

1. A system (102) for providing to a patient (104) information concerning a medical professional entering an accompanying patient room (106), comprising
- a detector (108) configured for detecting an entry of the medical professional in the patient room,
- a computer (110) for retrieving information concerning the medical professional from an organized collection of data (112) in response to detecting the entry of said medical professional in the patient room by the detector, and
- an electronic display (114) for displaying to the patient said information.

2. System according to claim 1 wherein the detector (108) is furthermore configured for detecting an exit of the medical professional from the patient room (106), and wherein the electronic display (114) is configured for ceasing displaying the information concerning said medical professional in response to detecting the exit of said medical professional from the patient room by the detector.

3. System according to claim 1 wherein the electronic display (114) is configured for simultaneously displaying information concerning a plurality of medical professionals in response to detecting the entries of said plurality of medical professionals in the patient room (106) by the detector (108).

4. System according to claim 3 wherein the electronic display (114) is configured for displaying information concerning the plurality of medical professionals in an arrangement according to an order of detecting the entries of said plurality of medical professionals in the patient room by the detector.

5. System according to claim 1 wherein the electronic display (114) is configured for displaying the name of the medical professional.

6. System according to claim 1 wherein the electronic display (114) is configured for displaying information relating to the role of the medical professional.

7. System according to claim 1 wherein the electronic display (114) is configured for displaying a photograph of the medical professional.

8. System according to claim 1, furthermore comprising a data storage (116) for storing the information concerning the medical professional, and a user interface for accessing the information stored by the data storage.

9. System according to claim 8 wherein the data storage (116) is configured for storing a timestamp along with the information concerning the medical professional.
